(19) Europäisches Patentamt
European Patent Office
Office européen des brevets

(11) Veröffentlichungsnummer: **0 025 528**
**B1**

# (12) EUROPÄISCHE PATENTSCHRIFT

(45) Veröffentlichungstag der Patentschrift: **29.12.82**

(51) Int. Cl.³: **C 07 C 149/42,** A 61 K 31/13

(21) Anmeldenummer: **80105102.0**

(22) Anmeldetag: **27.08.80**

(54) **1-(2-Acetylphenylthio)-3-alkylamino-2-propanole und deren Säureadditionssalze, Verfahren zu deren Herstellung und diese enthaltende Arzneimittel.**

(30) Priorität: **05.09.79 DE 2935901**

(43) Veröffentlichungstag der Anmeldung: **25.03.81 Patentblatt 81/12**

(45) Bekanntmachung des Hinweises auf die Patenterteilung: **29.12.82 Patentblatt 82/52**

(84) Benannte Vertragsstaaten: **AT BE CH FR GB IT LI LU NL SE**

(56) Entgegenhaltungen:
**DE-A-1 493 848**
**DE-B-1 236 523**
**Il Farmaco, Ed. Sc., Band 24, 1969, Seiten 349–357, Pavia, L. Villa et al.: »1-Isopropilamino-2-ossi-3-fenossipropani e composti correlati ad attivita beta-adreno-litica«**

(73) Patentinhaber: **A. Nattermann & Cie. GmbH, Nattermannallee 1, D-5000 Köln 30 (DE)**

(72) Erfinder: **Betzing, Hans, Dr., Heidstock 7, D-5014 Kerpen-Horrem (DE)**
Erfinder: **Uhlendorf, Joachim, Dr., Am Beissel 14, D-5042 Erftstadt-Lechenich (DE)**
Erfinder: **Gabbar, Hamied, Dr., Akazienweg 6, D-5042 Erftstadt-Lechenich (DE)**
Erfinder: **Graf, Erich, Dr., Am Langen Han 25, D-5014 Kerpen-Horrem (DE)**
Erfinder: **Doppelfeld, Ille-Stephanie, Im Bruchfeldchen 35, D-5151 Bergheim-Glessen (DE)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

EP 0 025 528 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

1-(2-Acetylphenylthio)-3-alkylamino-2-propanole und deren Säureadditionssalze, Verfahren zu deren Herstellung und diese enthaltende Arzneimittel

Gegenstand der Erfindung sind neue 1-(2-Acetylphenylthio)-3-alkylamino-2-propanole der allgemeinen Formel I

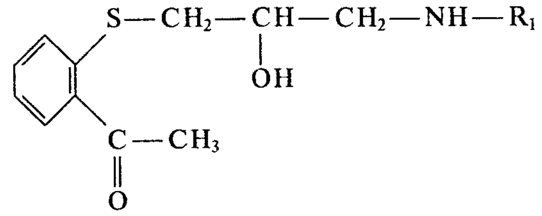

(I)

in der $R_1$ eine gerad- oder verzweigtkettige $C_1$ bis $C_5$-Alkyl- oder Cycloalkylgruppe bedeutet sowie ihre physiologisch verträglichen Säureadditionssalze, die $\beta$-sympatholytische Eigenschaften besitzen, weshalb sie sich zur Behandlung und Prophylaxe von Herzerkrankungen und zur Bekämpfung der Hypertonie eignen.

Die neuen Verbindungen der Formel I können in verschiedener Weise hergestellt werden. Ein besonders geeignetes Verfahren besteht in der Umsetzung eines Arylglycidsulfids der allgemeinen Formel II

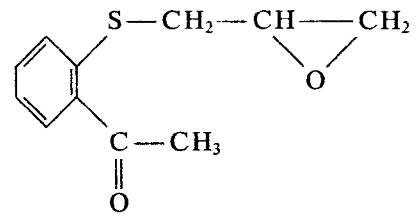

(II)

mit einem Alkylamin der allgemeinen Formel $H_2N-R_1$, wobei der Rest $R_1$ die Bedeutung wie in Formel I hat. Bevorzugte Lösungsmittel sind niedere Alkohole, insbesondere Äthanol oder Isopropanol, und cyclische Äther wie Tetrahydrofuran oder Dioxan, wobei die Umsetzung bei Temperaturen von 70—120° C und Normaldruck durchgeführt wird.

Eine weitere Art der Herstellung besteht in der Reaktion eines Halogenhydrins der allgemeinen Formel III

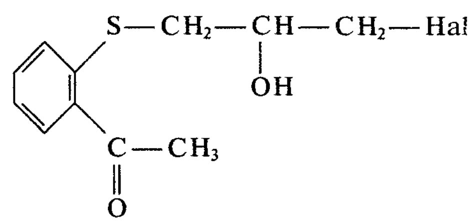

(III)

mit einem Amin der allgemeinen Formel $H_2N-R_1$. Zweckmäßigerweise wird die Umsetzung in Gegenwart oder Abwesenheit eines Lösungsmittels bei höheren Temperaturen unter Atmosphärendruck oder unter Druck in einem geschlossenen Gefäß, gegebenenfalls in Gegenwart eines säurebindenden Mittels, durchgeführt. Bevorzugte Lösungsmittel sind Äthanol oder Isopropanol.

Gemäß einer weiteren Herstellungsweise können die neuen Stoffe auch durch Kondensation eines Thiophenols der allgemeinen Formel IV

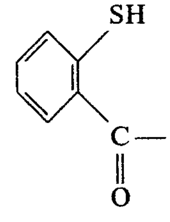

(IV)

mit einem 1-Halogeno-2-hydroxy-3-alkylaminopropan der allgemeinen Formel $X-CH_2-CHOH-CH_2-NH-R_1$, in der X für ein Halogenatom steht, erhalten werden. Die Reaktion wird in Gegenwart eines Lösungsmittels und eines säurebindenden Mittels bei Temperaturen von 70—120°C unter Inertgas ausgeführt.

Eine Variante dieser Methode ist die Umsetzung eines Disulfids der allgemeinen Formel V

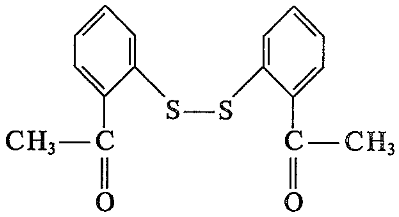

(V)

unter reduzierenden Bedingungen mit den angegebenen Reaktionspartnern. Als Reduktionsmittel werden hierbei bevorzugt Natriumborhydrid, Natriumdithionit oder Alkalisulfide in alkoholischer oder wäßriger Lösung verwendet.

Die Verbindungen I können auch hergestellt werden durch Umsetzung eines Aminoderivates der Formel VI

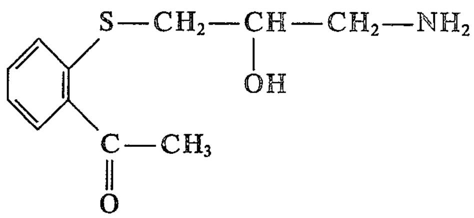

(VI)

mit der Verbindung der Formel $Z-R_1$ (worin Z = Chlor, Brom, Jod oder das Toluol-p-sulfonyloxy-Radikal bedeutet). Die Reaktion wird vorzugsweise in Gegenwart einer Base, wie z. B. Natrium- oder Kaliumcarbonat in einem Lösungsmittel, wie z. B. Äthanol oder Isopropanol, bei erhöhter Temperatur ausgeführt. Die Reinigung der erhaltenen erfindungsgemäßen Verbindungen erfolgt in üblicher Weise, beispielsweise durch Umkristallisieren aus einem Lösungsmittel, Überführen in eine Säureadditionsverbindung oder durch Säulenchromatographie.

Die erfindungsgemäßen Verbindungen der allgemeinen Formel I enthalten ein asymmetrisches Kohlenstoffatom in 2-Stellung der aliphatischen Seitenkette und werden als Racemate erhalten, die durch bekannte Methoden, beispielsweise über die Bildung diastereomerer Salze mit optisch aktiven Hilfssäuren, wie Weinsäure oder Campher-10-sulfonsäure, und deren fraktionierter Kristallisation in die optischen Antipoden getrennt werden können.

Von den Verbindungen der Formel I können in der üblichen Weise mit physiologisch verträglichen Säuren Säureadditionssalze hergestellt werden. Geeignete Säuren sind z. B. Salzsäure, Schwefelsäure, Phosphorsäure, Essigsäure, Milchsäure, Maleinsäure oder Fumarsäure.

Die bisher nicht beschriebenen Verbindungen der Formel I und ihre physiologisch verträglichen Säureadditionssalze besitzen wertvolle pharmakologische Eigenschaften. Sie können zur Behandlung und Prophylaxe bei Herz- und Kreislauferkrankungen verwendet werden.

Aus der DE-AS 12 36 523 war bekannt, daß bestimmte Phenoxypropyläther anipyretische, analgetische und lokalanästhetische Eigenschaften besitzen. Auch ist dort bereits beschrieben, daß basische 1-Phenyl- oder 1-Naphthyläther und -thioäther des 2-Hydroxy-3-tert.-butylaminopropans coronardilatorische Eigenschaften in Verbindung mit einer guten sedativen Wirkung besitzen. Auch in der DE-OS 14 93 848 ist offenbart, daß derartige 1-Phenyläther oder -thioäther des 2-Hydroxy-3-Aminopropans mit substituierter Aminogruppe, die gegebenenfalls am Phenylrest durch niedere Alkylreste oder Halogenatome substituiert sein können, zur Behandlung oder Prophylaxe von Herzkranzarterienerkrankungen brauchbar sind. In der Veröffentlichung Il Formaco Ed. Sc., Bd. 24, 1969, S. 349—357 wird von L. Villa et al berichtet, daß Phenoxy-hydroxyisopropylaminopropan $\beta$-adrenolytische Eigenschaften besitzt. Es ist aber überraschend, daß durch eine Substitution an der Phenylthioäthergruppe in 2-Stellung mit einem Acetylrest eine besonders starke und kompetitive $\beta$-rezeptorenblockierende Wirkung erreicht wird. Wegen der besonders deutlichen $\beta$-sympatholytischen Eigenaktivität eignen sich diese neuen Verbindungen besonders zur Behandlung von coronaren Herzerkrankungen, Herzrhythmusstörungen und der Hypertonie.

Die Wirksamkeit der erfindungsgemäßen Verbindungen als $\beta$-Blocker wurde durch die folgenden Tests im Vergleich zu der bekanntesten in der Praxis eingeführten Verbindung dieser Substanzgruppe »Propranolol« sowie zum Practolol belegt:

## Versuch 1

Es wurde in vitro an rechten, spontan schlagenden, und linken, elektrisch gereizten Meerschweinchen-Vorhöfen (Pirbright White) die direkte Wirkung auf Schlagfrequenz und Kontraktionskraft sowie die Wirkung auf die durch Isoprenalin ausgelöste Steigerung beider

Parameter untersucht. Die Präparation der Vorhöre erfolgte nach Holz und Westermann (Exp. Pathol, Pharmacol. 225, 421 [1955], die Versuchsanordnung nach Wagner et al (Arzneim. Forsch. 22, 1061 [1972]). Als $ED_{50}$-Werte wurden diejenigen Konzentrationen der erfindungsgemäßen Verbindung ermittelt, die den Isoprenalin-Effekt ($5,8 \cdot 10^{-9}$ molar bei rechten und $3,2 \cdot 10^{-8}$ molar bei linken Vorhöfen) auf die Hälfte reduzierten. Die ermittelten Ergebnisse sind in der Tabelle aufgeführt.

Versuch 2

Zum Nachweis des kompetitiven $\beta$-adrenergischen Antagonismus in vivo wurden mischrassige Katzen beiderlei Geschlechts und in Chloralose-Pentobarbitalnarkose verwendet. Nach der Methode von Åblad et al (Eur. J. Pharmacol. 13, 59 [1970]) wurde zur Messung des Blutdruckablaufs ein Polyäthylenkatheter in die rechte Arteria femoralis eingeführt. Die Registrierung erfolgte über ein Stathamelement aus einem Beckman Dynograph Typ R. Aus der Blutdruckamplitude wurde mit Hilfe eines Kardiotachometers die Herzfrequenz abgenommen und auf einem weiteren Kanal des Dynographen aufgezeichnet. Die Applikation der Testlösungen erfolgte über einen Katheter in die linke Vena femoralis. Es wurde die inhibierende Wirkung ansteigender Testsubstanz-Dosen auf die durch i. v.-Gabe von 0,5 µg Isoprenalin/kg Körpergewicht induzierte Tachykardie bzw. Hypotonie (Blutdrucksenkung) geprüft.

Als $ED_{50}$-Werte wurden in der Tabelle die Dosen der Referenz- bzw. erfindungsgemäßen Verbindung eingetragen, die die Isoprenalineffekte jeweils auf die Hälfte reduzierten.

Versuch 3

Die sympathomimetische Eigenaktivität (ISA) wurde nach der Methode von Barret & Carter (Br. J. Pharmacol. 40, 373 [1970]) an pentobarbitalnarkotisierten, männlichen Wistar-Ratten durchgeführt. Die Tiere bekamen 24 Stunden vor Versuchsbeginn 5 mg Reserpin/kg intraperitoneal. Die Herzfrequenz wurde aus der Blutdruckamplitude (Polyäthylenkatheter mit aufgesetztem Stathamelement in der rechten Arteria femoralis) mit Hilfe eines Kardiotachometers abgenommen und auf einen Schreiber fortlaufend registriert. Die Applikation der Testlösungen erfolgte über einen Katheter in der linken Vena femoralis. Es wurden von jeder Testverbindung vier ansteigende Dosen getrennt jeweils an sechs verschiedenen Tieren geprüft.

Die maximale Steigerung der Herzfrequenz (Mittelwert aus 6 Tieren) durch die einzelnen Testverbindungen wurde als Maß für ihre agonistische Aktivität genommen und in Prozent der Frequenzsteigerung durch 1,0 µg Isoprenalin/kg Körpergewicht i. v. errechnet.

Die ermittelten Ergebnisse sind aus der Tabelle zu entnehmen.

Versuch 4

Die akute Toxizität wurde an NMRI-Mäusen durch intravenöse bzw. orale Gabe von steigenden Dosen der erfindungsgemäßen Verbindung ermittelt. Die Todesraten wurden innerhalb der 7tägigen Beobachtung festgestellt und die mittlere letale Dosis ermittelt.

Die in der Tabelle beispielhaft aufgeführten Ergebnisse zeigen, daß die erfindungsgemäßen Verbindungen sowohl am isolierten Organ als auch am Ganztier eine starke und kompetitive $\beta$-rezeptorenblockierende Wirkung besitzen und somit einen therapeutisch nützlichen Schutz vor einem pathologisch erhöhten Sympathikotonus liefern. Der Vergleich mit zwei bekannten Referenzsubstanzen dieser Indikationsklasse kann diese Aussage verdeutlichen, wobei darauf hingewiesen wird, daß Propanolol das wichtigste und am häufigsten benutzte Arzneimittel dieser Indikationsgruppe darstellt. Besonders zu erwähnen ist die ausgeprägte sympathomimetische Eigenaktivität der Verbindungen, die bekanntlich dem Propanolol fehlt.

Ergebnisse der pharmakologischen und toxikologischen Untersuchungen der erfindungsgemäßen Verbindung im Vergleich zu zwei Referenzsubstanzen.

| Versuch | | Applikation | Propanolol | Practolol | Erfindungsgemäße Verbindung[1]) |
|---|---|---|---|---|---|
| 1. | Isolierter Meerschweinchen-Vorhof: | | $ED^*_{50}$ | $ED^*_{50}$ | $ED^*_{50}$ |
| 1.1. | Hemmung der Isoprenalin-induzierten Frequenzsteigerung | Organbad | $1{,}5 \cdot 10^{-7}$ molar | $7{,}1 \cdot 10^{-7}$ molar | $1{,}5 \cdot 10^{-7}$ molar |
| 1.2. | Hemmung der Isoprenalin-induzierten Kontraktionskraftsteigerung | Organbad | $4{,}5 \cdot 10^{-7}$ molar | $2{,}3 \cdot 10^{-6}$ molar | $4{,}7 \cdot 10^{-7}$ molar |
| 2. | Narkotisierte Katze: | | $ED^*_{50}$ | $ED^*_{50}$ | $ED^*_{50}$ |
| 2.1. | Hemmung der Isoprenalin-induzierten Herzfrequenzsteigerung | intravenös | 0,017 mg/kg | 0,144 mg/kg | 0,044 mg/kg |
| 3. | Reserpinisierte Ratte (in Narkose) | | in % der sympathomimetischen Aktivität von Isoprenalin | | |
| 3.1. | β-sympathomimetische Eigenaktivität (ISA) | intravenös | 0% | 54% | 33% |
| 4. | Akute Toxizität | | $LD^*_{50}$ | $LD^*_{50}$ | $LD^*_{50}$ |
| 4.1. | Maus | intravenös | 40 mg/kg | 148 mg/kg | 111 mg/kg |
| 4.2. | Maus | oral | 280 mg/kg | 2,874 mg/kg | 667 mg/kg |
| 5. | ›Therapeutische Breite‹ Quotient von 4.1. : 2.1. | | 2353 | 1028 | 2523 |

[1]) Erfindungsgemäße Verbindung: 1-(2-Acetylphenylthio)-tert.-butylamino-2-propanol · HCl.
* Ermittelt mit Hilfe der Probitanalyse.

Die erfindungsgemäßen Verbindungen können in den üblichen Anwendungsformen allein oder in Kombination mit anderen Wirkstoffen, wie z. B. Antihypertonika, Diuretika, Thrombozytenaggregationshemmern oder Coronardilatoren, verabreicht werden.

Die therapeutischen Zubereitungen werden mit den üblichen Trägerstoffen oder Verdünnungsmitteln und üblicherweise verwendeten pharmazeutisch-technischen Hilfsstoffen je nach Applikationsart mit einer geeigneten Dosierung in an sich bekannter Weise hergestellt. Die bevorzugten Zubereitungen bestehen in einer Darreichungsform, die zur oralen Applikation geeignet ist. Solche Darreichungsformen sind z. B. Tabletten, Filmtabletten, Dragees, Kapseln, Pillen oder Depotformen. Auch parenterale Zubereitungen, wie Injektionslösungen, kommen in Betracht.

Die erfindungsgemäßen Verbindungen werden durch die folgenden Beispiele näher beschrieben.

Die als Ausgangsprodukte verwendeten Thiophenole werden, wenn nicht besonders erwähnt, durch Reduktion der entsprechenden Sulfonsäurechloride oder nach Leuckart via Diazoniumsalz und Xanthogenat aus den Anilinen hergestellt.

## Beispiel 1

Zu einer rührenden Lösung von 15,1 g (0,1 Mol) o-Mercaptoacetophenon in 100 ml Wasser und 8 g Natriumhydroxyd wird bei Raumtemperatur und unter Stickstoff 11,2 g (0,12 Mol) Epichlorhydrin zugetropft. Es scheidet sich sofort ein Öl ab. Nach zweistündigem Rühren wird das Gemisch aus Epoxyd und Chlorhydrin durch Extraktion mit 100 ml Chloroform abgetrennt, mit wasserfreiem

Natriumsulfat getrocknet und eingeengt. Man erhält 19,6 g eines bläulichen Öls, welches ohne Reinigung weiter umgesetzt wird.

19,6 g der erhaltenen Gemische, 20 ml tert.-Butylamin und 50 ml Isopropanolol werden 15 Stunden unter Rückfluß erhitzt. Nach dem Eindampfen im Vakuum werden 25 g eines braunen Öls gewonnen. Es wird in 120 ml Chloroform gelöst und dreimal mit je 50 ml 4n HCL extrahiert. Die wäßrige Phase wird alkalisch gestellt und zweimal mit je 100 ml Essigester ausgeschüttelt. Die vereinigten Extrakte werden gewaschen, getrocknet und unter reduziertem Druck eingeengt. Es wird 15 g 1-(2'-Acetylphenylthio)-3-tert.-butylamino-2-propanol als bräunliches Öl erhalten. Die Base, in Isopropanol gelöst, wird durch tropfenweisen Zusatz von ätherischer HCL zur Kristallisation gebracht. Das Salz wird abgesaugt, nochmaliges Umkristallisieren aus Isopropanol liefert das reine Hydrochlorid.

Ausbeute: 11,5 g, Fp: 156–8°C.

Die folgenden Verbindungen werden nach der Methodik des Beispiels 1 hergestellt:

| Beispiele | $R_1$ | Fp (HCl) |
|---|---|---|
| 2 | Isopropyl | 182-3° |
| 3 | Cyclopentyl | 180-2° |
| 4 | sek.-Butyl | 125-7° |
| 5 | Cyclopropyl | 183-6° |

**Patentansprüche für die Vertragsstaaten BE, CH, LI, FR, GB, IT, LU, NL, SE**

1. 1-Phenylthio-3-alkylamino-2-propanole der allgemeinen Formel I

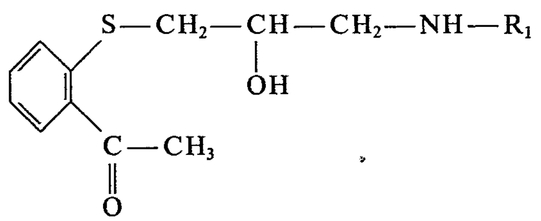

(I)

in der $R_1$ eine gerad- oder verzweigtkettige $C_1$- bis $C_5$-Alkyl- oder Cycloalkylgruppe bedeutet und ihre physiologisch verträglichen Säureadditionssalze.

2. Verfahren zur Herstellung der Verbindungen der Formel I gemäß Anspruch 1, dadurch gekennzeichnet, daß man in an sich bekannter Weise

a) ein Amin der Formel $H_2N-R_1$ mit einem Arylglycidsulfid der allgemeinen Formel II

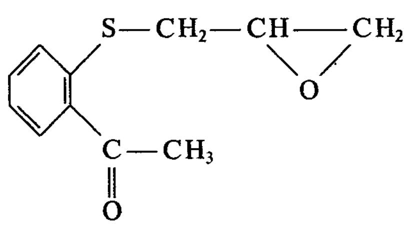

(II)

oder mit einem Halogenhydrin der allgemeinen Formel III

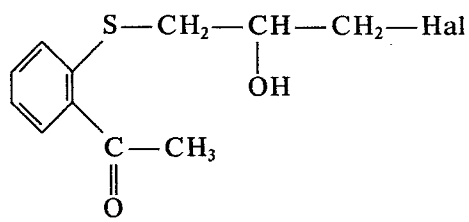

(III)

worin Hal für Chlor, Brom oder Jod steht, umsetzt, oder

b) ein Thiophenol der allgemeinen Formel IV

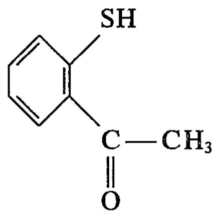

(IV)

mit einer Verbindung der Formel $X-CH_2-NH-R_1$, worin X für die

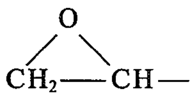

oder $Y-CH_2-CH(OH)$-Gruppe (worin Y ein ersetzbares Radikal bedeutet) steht, umsetzt, oder

c) ein Disulfid der allgemeinen Formel V

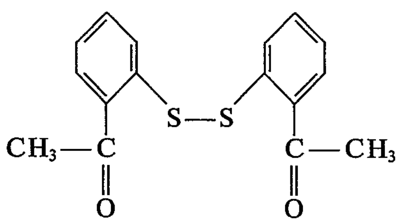

(V)

unter reduzierenden Bedingungen mit den unter b) angegebenen Partnern zur Reaktion bringt, oder

d) ein Aminoderivat der Formel VI

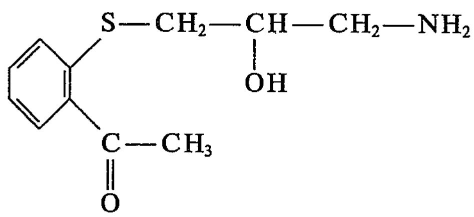

(VI)

mit einem Alkylhalogenid der Formel $Hal-R_1$ reagieren läßt, wobei $R_1$ die gleiche Bedeutung hat wie in der allgemeinen Formel I.

3. Arzneimittel, gekennzeichnet durch den Gehalt an einer Verbindung nach Anspruch 1 neben üblichen Trägerstoffen und Verdünnungsmitteln.

**Patentanspruch für den Vertragsstaat AT**

Verfahren zur Herstellung der 1-Phenylthio-3-alkyl-amino-2-propanole der allgemeinen Formel I

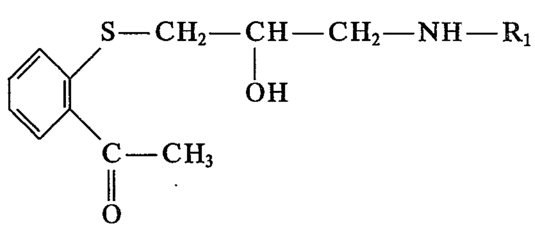

(I)

in der $R_1$ eine gerad- oder verzweigtkettige $C_1$- bis $C_5$-Alkyl- oder Cycloalkylgruppe bedeutet und ihre physiologisch verträglichen Säureadditionssalze, dadurch gekennzeichnet, daß man in an sich bekannter Weise

a) ein Amin der Formel $H_2N-R_1$ mit einem Arylglycidsulfid der allgemeinen Formel II

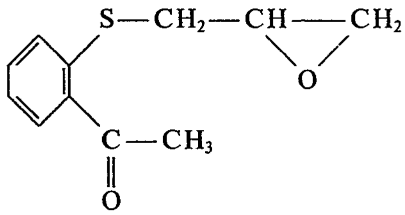

(II)

oder mit einem Halogenhydrin der allgemeinen Formel III

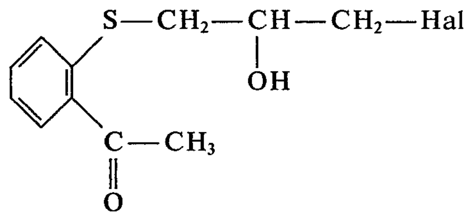

(III)

worin Hal für Chlor, Brom oder Jod steht, umsetzt, oder

b) ein Thiophenol der allgemeinen Formel IV

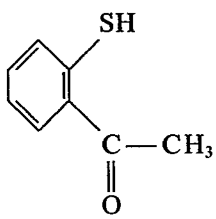

(IV)

mit einer Verbindung der Formel $X-CH_2-NH-R_1$, worin X für die

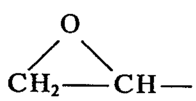

oder $Y-CH_2-CH(OH)$-Gruppe (worin Y ein ersetzbares Radikal bedeutet) steht, umsetzt, oder

c) ein Disulfid der allgemeinen Formel V

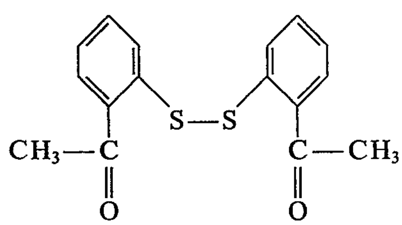

(V)

unter reduzierenden Bedingungen mit den unter b) angegebenen Partnern zur Reaktion bringt, oder

d) ein Aminoderivat der Formel VI

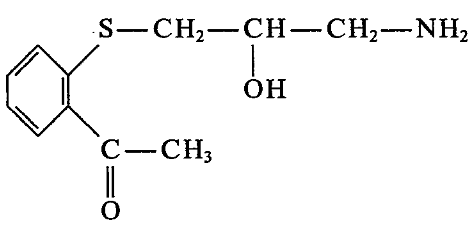

(VI)

mit einem Alkylhalogenid der Formel $Hal-R_1$ reagieren läßt, wobei $R_1$ die gleiche Bedeutung hat wie in der allgemeinen Formel I.

**Claims for the contracting states BE, CH, LI, FR, GB, IT, LU, NL, SE**

1. 1-phenylthio-3-alkylamino-2-propanoles represented by the general formula I

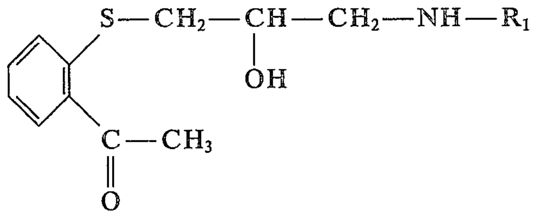

(I)

in which $R_1$ is a straight chain or a branched $C_1$ to $C_5$-alkyl or cycloalkyl group, and the physiologically acceptable acid addition salts thereof.

2. A process for the production of the compounds of formula I in accordance with claim 1 characterized in that, in a per se known manner,

a) an amine of the formula $H_2N-R_1$ is reacted with an arylglycidsulfide represented by the general formula II

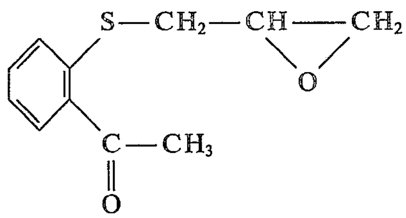

(II)

or with a halogen hydrine represented by the general formula III

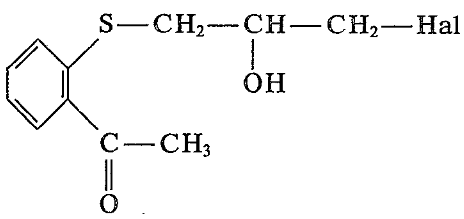

(III)

wherein Hal is chlorine, bromine or iodine, or

b) a thiophenole represented by the general formula IV

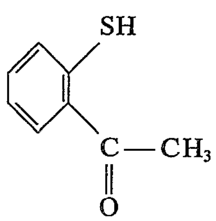

(IV)

is reacted with a compound of the formula $X-CH_2-NH-R_1$, wherein X represents the

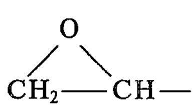

or $Y-CH_2-CH(OH)$ group, wherein Y is a replaceable radical, or

c) a disulfide represented by the general formula V

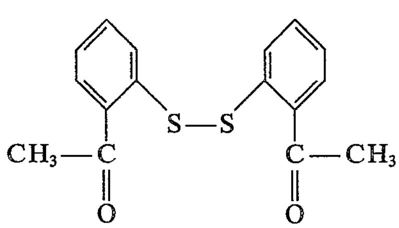

(V)

is under reducing conditions reacted with the partners indicated under (b), or

d) an amino derivative of the formula VI

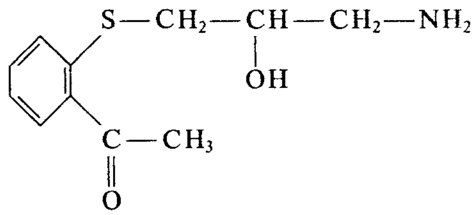

(VI)

is allowed to react with an alkylhalogenide of the formula $Hal-R_1$, wherein $R_1$ has the same meaning as in the general formula I.

3. A pharmaceutical preparation characterized by the content of a compound as claimed in claim 1 in addition to common carriers and diluents.

**Claim for the contracting state AT**

A process for the production of 1-phenylthio-3-alkyl-amino-2-propanoles represented by the general formula I

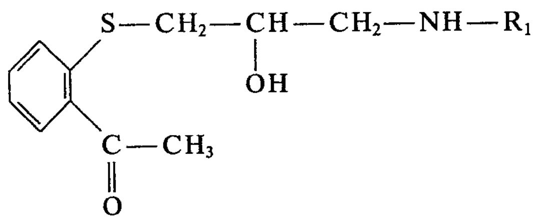

(I)

in which $R_1$ is a straight chain or a branched $C_1$ to $C_5$ alkyl or cycloalkyl group, and its physiologically acceptable acid addition salts characterized in that, in a per se known manner,

(a) an amine of the formula $H_2N-R_1$ is reacted with an arylglycidsulfide represented by the general formula II

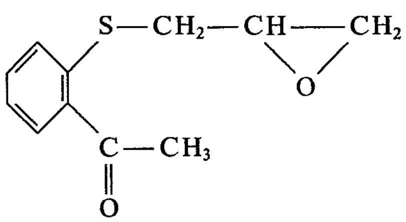

(II)

or with a halogen hydrine represented by the general formula III

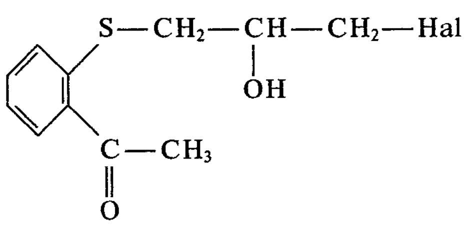

(III)

wherein Hal is chlorine, bromine or iodine, or

b) a thiophenole represented by the general formula IV

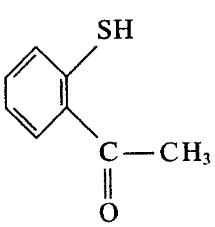

(IV)

is reacted with a compound of the formula $X-CH_2-NH-R_1$, wherein X represents the

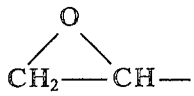

or $Y-CH_2-CH(OH)$ group, wherein Y is a replaceable radical, or

(c) a disulfide represented by the general formula V

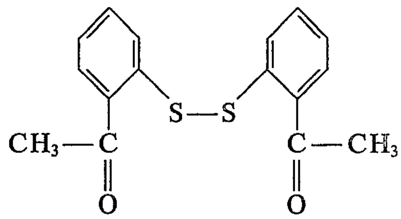

(V)

is under reducing conditions reacted with the partners indicated under (b), or

(d) an amino derivative of the formula VI

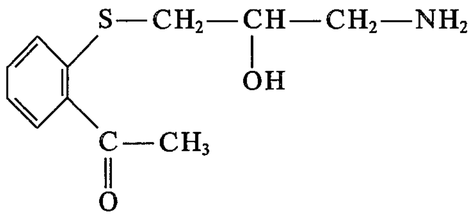

(VI)

is allowed to react with an alkylhalogenide of the formula $Hal-R_1$, wherein $R_1$ has the same meaning as in the general formula I.

**Revendications pour les Etats contractans BE, CH, LI, FR, GB, IT, LU, NL, SE**

1. 1-phénylthio-3-alcoylamino-2-propanols de formule générale I

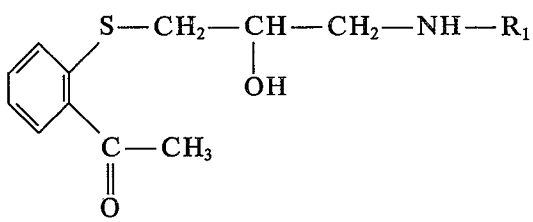

(I)

où $R_1$ représente un groupe alcoyle en $C_1$ à $C_5$ à chaîne droite ou ramifiée ou un groupe cycloalcoyle, et leurs sels d'addition d'acides physiologiquement acceptables.

2. Procédé de préparation des composés de formule I selon la revendication 1, caractérisé en ce que de façon classique

a) on fait réagir une amine de formule $H_2N-R_1$ avec un sulfure d'arylglycide de formule générale II

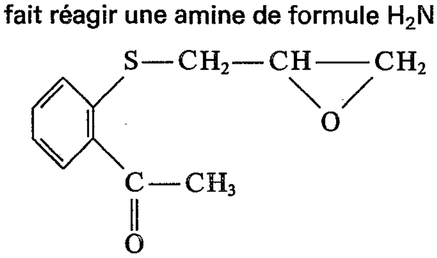

(II)

ou avec une halogénhydrine de formule générale III

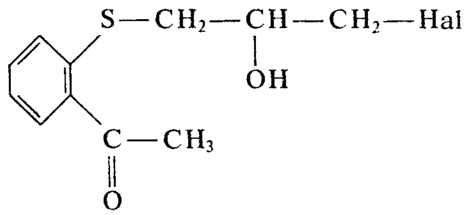

(III)

où Hal représente un atome de chlore, de brome ou d'iode, ou

b) on fait réagier un thiophénol de formule générale IV

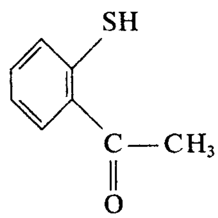

(IV)

avec un composé de formule $X-CH_2-NH-R_1$, où X représente le groupe

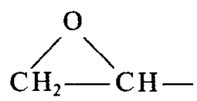

ou $Y-CH_2-CH(OH)$ (où Y représente un radical remplaçable) ou

c) on fait réagir un disulfure de formule générale V

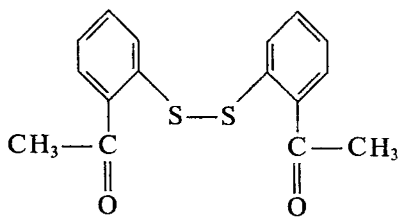

(V)

dans des conditions réductrices avec les réactifs mentionnés en b), ou

d) on fait réagir un dérivé aminé de formule VI

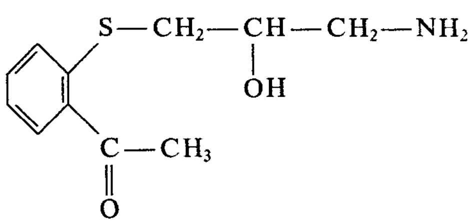

(VI)

avec un halogénure d'alcoyle de formule $Hal-R_1$, où $R_1$ a la même signification que dans la formule générale I;

3. Médicament caractérisé en ce qu'il contient un composé selon la revendication 1, outre les supports et diluants habituels.

**Revendication pour l'Etat contractant AT**

Procédé de préparation des 1-phénylthio-3-alcoylamino-2-propanols de formule générale I

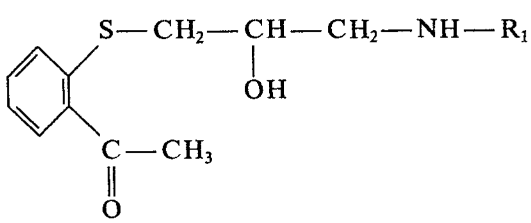

(I)

où $R_1$ représente un groupe alcoyle en $C_1$ à $C_5$ à chaîne droite ou ramifiée ou un groupe cycloalcoyle, et de leurs sels d'addition d'acides physiologiquement acceptables, caractérisé en ce que de façon classique

a) on fait réagier une amine de formule $H_2N-R_1$ avec un sulfure d'arylglycide de formule générale II

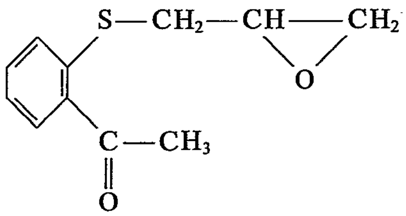

(II)

ou avec une halogénhydrine de formule générale III

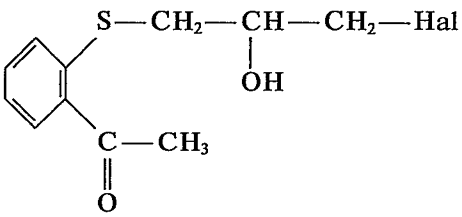

(III)

où Hal représente un atome de chlore, de brome ou d'iode, ou

b) on fait réagir un thiophénol de formule générale IV

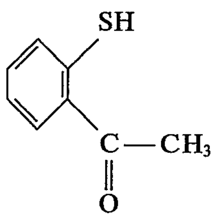

(IV)

avec un composé de formule $X-CH_2-NH-R_1$, où X représente le groupe

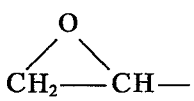

ou $Y-CH_2-CH(OH)$ (où Y représente un radical remplaçable), ou

c) on fait réagier un disulfure de formule générale V

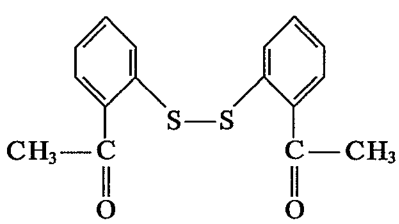

(V)

dans des conditions réductrices avec les réactifs mentionnés en b), ou

d) on fait réagier un dérivé aminé de formule VI

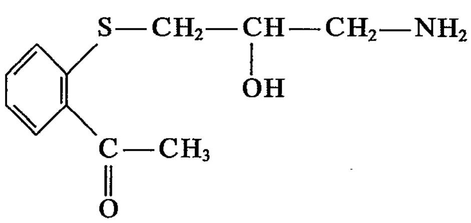

(VI)

avec un halogénure d'alcoyle de formule $Hal-R_1$, où $R_1$ a la même signification que dans la formule générale I.